(19) Europäisches Patentamt
European Patent Office
Office européen des brevets

(11) **EP 1 884 620 B1**

(12) **FASCICULE DE BREVET EUROPEEN**

(45) Date de publication et mention
de la délivrance du brevet:
**02.12.2009 Bulletin 2009/49**

(51) Int Cl.:
***E21B 49/00*** (2006.01)

(21) Numéro de dépôt: **07290893.2**

(22) Date de dépôt: **17.07.2007**

(54) **Méthode pour quantifier la formation et la rétention d'hydrocarbures dans une roche mère**

Verfahren zur Quantifizierung der Bildung und Retention von Kohlenwasserstoffen in Muttergestein

Method of quantifying the formation and retention of hydrocarbons in source rock

(84) Etats contractants désignés:
**DE GB**

(30) Priorité: **04.08.2006 FR 0607148**

(43) Date de publication de la demande:
**06.02.2008 Bulletin 2008/06**

(73) Titulaire: **IFP**
**92852 Rueil-Malmaison Cédex (FR)**

(72) Inventeurs:
• **Salmon, Elodie**
**13400 Aubagne (FR)**
• **Lorant, Francois**
**94320 Thiais (FR)**
• **Behar, Francoise**
**75007 Paris (FR)**

(56) Documents cités:
• **S. R. KELEMEN, C. C. WALTERS, D. ERTAS, H. FREUND , D. J. CURRY: "Petroleum Expulsion Part 3. A Model of Chemically Driven Fractionation during Expulsion of Petroleum from Kerogen" ENERGY FUELS, [Online] no. 20, 5 janvier 2006 (2006-01-05), pages 309-319, XP002427301 Online Extrait de l'Internet: URL: http://pubs.acs.org/cgi-bin/abstract.c gi/enfuem/ 2006/20/i01/abs/ef058023s.html> [extrait le 2007-03-29]**
• **ULRICH RITTER, AND ARNT GRØVER: "Adsorption of petroleum compounds in vitrinite: implications for petroleum expulsion from coal" INTERNATIONAL JOURNAL OF COAL GEOLOGY, vol. 62, no. 3, 11 mai 2005 (2005-05-11), pages 183-191, XP002427302 Trondheim**
• **STANISLAV I. STOLIAROVA, RICHARD E. LYONB AND MARC R. NYDEN: "A reactive molecular dynamics model of thermal decomposition in polymers. II. Polyisobutylene" POLYMER, vol. 45, no. 25, 30 novembre 2004 (2004-11-30), pages 8613-8621, XP002427303 Online**
• **ADRI VAN DUIN: "ReaxFF reactive force field: a new link from QM to MM" CMDF WORKSHOP, [Online] 23 août 2005 (2005-08-23), pages 1-21, XP002427304 California Extrait de l'Internet: URL:http://www.wag.caltech.edu/events/cmdf /CMDFworkshop_duin.pdf> [extrait le 2007-03-29]**

## Description

La présente invention concerne une méthode pour quantifier la formation et la rétention d'hydrocarbures au sein d'un système chimique macromoléculaire.

La présente invention comporte une méthode servant à modéliser la réactivité thermique de systèmes chimiques macromoléculaires dont la structure moléculaire est inconnue ou mal connue.

En particulier, on peut l'utiliser dans le cadre des modélisations de bassin où la méthode permet de paramétrer la proportion d'hydrocarbures formés et éventuellement retenus dans la matrice organique de la roche mère.

Dans la description qui suit, on désigne par "système chimique macromoléculaire" ou "système macromoléculaire" un système formé d'au moins une macromolécule organique, par exemple la matière organique de la roche-mère.

On entend par "schéma réactionnel" l'ensemble des réactions chimiques décrivant la maturation thermique de chaque macromolécule. Un schéma réactionnel est associé à un ensemble de données quantitatives et qualitatives caractérisant les réactifs, les produits et les vitesses des réactions chimiques.

On désigne par "champ de forces" un ensemble d'équations paramétrées décrivant les différentes contributions à l'énergie potentielle totale d'un système chimique (notamment énergies de Van der Waals, de Coulomb, de torsion).

## Présentation de l'art antérieur

Les documents suivants, cités dans le cours de la description ci-après, illustrent l'état de la technique :

- Behar F., Vandenbroucke M., Tang Y., Marquis F., Espitalié J., 1997. Thermal cracking of kerogen in open and closed systems: determination of kinetic parameters and stoichiometric coefficients for oil and gas generation. Org. Geochem., 26, 5-6, 321-339.
- Burnham, A. K. and Braun, R. L., 1989. Development of detailed model of petroleum formation, destruction, and expulsion from lacustrine and marine source rocks. Advances in Organic Geochemisrty, 16, 1-3, 27-39.
- Burnham, A. K. and Braun, R. L., 1990. Mathematical model of oil generation, degradation, and expulsion. Energy and Fuel, 4, 132-146.
- Faulon, J. L., Prediction Elucidation and Molecular Modeling. Algorithms and Applications in Geochemistry, Ph. D.Thesis, Edited by Ecole des Mines, Paris, 1991.
- Faulon, J. L., Stochastic Generator of Chemical Structure. (4) Building Polymeric Systems with Specified Properties, J. Comput. Chem., 2001, 22, 580-590.
- Freund, H., Walters, C. C., Kelemen, S. R., Siskin, M., Curry, D. J., Xiao, Y., Olmstead, W. N., Gorbaty, M. L., Bence, A. E., 2005. Predicting oil and gas compositional yields via chemical structure- chemical yield modeling (CS-CYM). Organic Geochemistry: Challenges for the 21st Century (Vol. 1), 22 IMOG Seville, Spain, 66-67.
- Hatcher, P. G.,. 1988.. Dipolar-Dephasing 13C studies of decomposed wood and coalified xylem tissue: Evidence for chemical structural changes associated with defunctionalization of lignin structural units during coalification..Energy & Fuels.. 2, 48-58.
- Pepper, A. S., 1991.Estimating the petroleum expulsion behaviour of source rocks: a novel quantitative approach. In Petroleum Migration (Edited by England W. A. and Feed A. J.), Geological Society, Special Publication.59, pp. 9-31.
- Pepper, A. S., Corvi, P. J., 1995. Simple kinetic models of petroleum formation. PartIII: Modelling and open system. Marin and Petroleum Geology, 12, 4, 417-452.
- Pepper, A. S., Dodd, T. A., 1995. Simple models of petroleum formation. Part II: oil to ges cracking. Mar. Petrol. Geol., 12, 321-340.
- Ritter, U., 2003. Fractionation of petroleum during expulsion from kerogen.Journal of Geochemical Exploration . 78-79, 417-420.
- Ritter, U., 2003.Solubility of petroleum compounds in kerogen: implications for petroleum expulsion. Organic Geochemistry .34, 319-326.
- Ritter, U., Groever, A. 2005. Adsorption of petroleum compounds in vitrinite: implications for petroleum expulsion from coal. International Journal of Coal Geology. Vol. 62, n° 3, 183-191.
- Tissot, B. 1969. Revue Inst. Fr. Pétrole, 24(4), 470-501.
- Ungerer, P., 1989. State of the art of research in kinetic modelling of oil formation and expulsion.In Advances in organic goechemistry, Organic Geochemistry .16, 1-3, 1-25.
- Van Duin A.C.T, Siddharth D., Lorant F., Goddard III W.A..-2001. ReaxFF: a reactive force field for hydrocarbons.. J. Phys. Chem. A, .105, 9396-9409.
- Van Duin A. ReaxFF reactive force field : a new link from QM to MM. 2005. CMDF WORKSHOP, 1-21.

La matière organique insoluble de la roche mère, également appelée kérogène, est un mélange de macromolécules bio organiques (notamment de biogéopolymères) possédant des structures chimiques aliphatiques et aromatiques qui évoluent au cours des temps géologiques avec la température et la pression. La maturation thermique du kérogène dans la roche mère se produit au moyen de deux phénomènes principaux :

- le premier est le craquage thermique de la matière organique à l'origine des hydrocarbures ; celui-ci a lieu naturellement dans les bassins sédimentaires, généralement à une température comprise entre 80 et 200°C, pour des pressions allant de 200 à 1000 bars ;

- le second est l'évolution physico-chimique des produits pétroliers au sein de la roche-mère expliquant la rétention et l'expulsion des hydrocarbures hors de la roche-mère.

Ces deux phénomènes évoluent dans le même contexte et se juxtaposent.

En modélisation de bassin, il est important de pouvoir calibrer simultanément les quantités d'hydrocarbures formés et les quantités d'hydrocarbures "libres" pouvant être expulsés de la roche mère et migrant jusqu'au réservoir. Le processus de rétention des hydrocarbures dans le kérogène constitue un mécanisme qui contrôle le ratio hydrocarbures libres/hydrocarbures expulsés. Si l'on considère que la rétention des hydrocarbures se déroule majoritairement dans la matière organique de la roche mère, cette rétention est dépendante de la composition des fluides générés, des capacités de rétention du kérogène et du rapport volumique entre la matière organique solide et les hydrocarbures liquides. Elle est donc directement liée à la nature physicochimique du kérogène et au taux de transformation (Pepper, 1991).

Les phénomènes de craquage du kérogène et de rétention des produits issus de cette réaction étant interdépendants et évoluant avec la maturation thermique du kérogène, il apparaît nécessaire de développer une analyse fine de la réaction de craquage couplée à la rétention des produits formés dans le kérogène afin de pouvoir estimer les hydrocarbures retenus dans le kérogène et les hydrocarbures disponibles au stockage dans les réservoirs.

On connaît des modèles de quantification des hydrocarbures prenant en compte soit la réaction de craquage thermique, soit le phénomène de rétention des hydrocarbures, mais rarement les deux phénomènes en même temps :

- les modèles de craquage empiriques et mécanistiques servent à quantifier les hydrocarbures formés dans la roche mère.

- les modèles de rétention des hydrocarbures dans la roche mère cherchent quant à eux à expliquer la ségrégation des hydrocarbures lorsqu'ils sont expulsés de la roche mère.

Modèles de craquage thermique:

Deux méthodes de modélisation de la réaction de craquage sont proposées dans la littérature : les modèles empiriques et les modèles mécanistiques.

Les modèles empiriques s'appuient sur l'expérience afin d'établir les équations stoechiométriques globales qui rendent compte des bilans massiques observés.

Ces équations stoechiométriques sont couplées à des lois de vitesse de formation des hydrocarbures, elles correspondent à une série de réactions simultanées, indépendantes et compétitives et ont été développées en supposant que l'évolution globale du potentiel pétrolier d'un kérogène en cours de maturation est un processus cinétique irréversible (Pepper et Dodd 1995). Les paramètres cinétiques (E : énergie d'activation, A : facteur de fréquence) et stoechiométriques (Xi : contribution relative de la réaction i) doivent être calibrés au cas par cas, car les roches mères ne génèrent pas les hydrocarbures à la même vitesse. Pour cela, on réalise des expériences de maturation artificielle, au laboratoire, dans des conditions thermiques contrôlées. Ces expériences sont effectuées sur des kérogènes ou des roches mères et peuvent être de natures différentes. Par inversion numérique des données de laboratoire, il est possible de calculer les paramètres cinétiques et stoechiométriques. Ces paramètres obtenus à haute température (300-600°C) sur des temps courts (quelques min à quelques jours) sont ensuite supposés extrapolables pour des températures inférieures à celles des conditions expérimentales telles que celles imposées par les gradients géothermiques.

Actuellement cette méthode est le seul moyen qui permette de donner des informations sur la formation des hydrocarbures compatibles avec les modèles de bassins. Toutefois cette démarche est basée sur de nombreuses approximations. En effet, des travaux de Behar et al. 1997 ont montré que les différences de conditions expérimentales des pyrolyses (milieu ouvert ou fermé, en présence ou absence d'eau ou de matrice minérale, ou suivant la granulométrie de l'échantillon) entraîne un décalage des paramètres cinétiques et donc une incertitude dans l'estimation de la fenêtre à huile. De la même façon l'extrapolation des paramètres cinétiques à basse température suppose que la nature du mécanisme de craquage n'évolue pas significativement en fonction de la température.

Les modèles mécanistiques s'appuient non pas sur des équations stoechiométriques, mais sur des réactions élémentaires (radicalaires) pour simuler la dégradation thermique de macromolécules complexes et reproduire la distribution des hydrocarbures formés (Freund et al. 2005). L'élaboration de ces modèles commence par la modélisation de la macromolécule de départ. Cette modélisation est contrainte par des données expérimentales portant sur les propriétés structurales de l'échantillon. Elle se base sur la distribution des groupements fonctionnels dans la molécule pour en établir la structure probable. Une fois que la macromolécule de départ est définie, des réactions élémentaires sont appliquées à la structure pour simuler la formation des produits de dégradation thermique. Chaque réaction élémentaire possède des propriétés cinétiques propres, valables quelle que soit l'échelle de température. De cette façon il est possible de simuler à la fois la maturation thermique dans les conditions de laboratoire ou dans les conditions géologiques.

L'avantage de cette approche est qu'elle minimise l'incertitude sur les vitesses de réaction extrapolées dans les conditions géologiques. En revanche, la complexité de ces modèles rend difficile l'élaboration du schéma réactionnel. Enfin et surtout, le nombre très élevé de réactions dans ces modèles est incompatible avec les simulateurs de bassin à ce jour.

Modèles de rétention:

Des modèles physiques ont été proposés dans le but d'estimer la proportion d'hydrocarbures expulsés en fonction du taux de transformation du kérogène. Le modèle d'expulsion de Ungerer (1989) propose de fixer un seuil correspondant à un taux de transformation pour lequel les hydrocarbures formés dans la roche mère sont expulsés. Dans ce même esprit, Pepper (1991) envisage de corréler "l'efficacité de l'expulsion du pétrole" (PPE) avec le potentiel pétrolier initial des roches mères considérées. Ces modèles reproduisent avec plus ou moins de fidélité (suivant le type de roche mère) la qualité des hydrocarbures du réservoir. Ils n'envisagent la roche mère que sous deux états, avant et après l'expulsion des hydrocarbures sans prendre en considération l'évolution qualitative de la roche mère ni la cinématique de l'expulsion.

Plus tard Ritter (2003) propose un modèle de rétention basé sur la solubilité des hydrocarbures dans le kérogène. Il établit une relation empirique entre le coefficient d'avalement volumique et le paramètre de solubilité de Hildebrand, pour chaque type de matière organique. Cette relation définit un taux de rétention pour chaque groupe de composés. Finalement ce modèle confirme la séquence de fractionnement observée dans la nature sauf pour les hydrocarbures aliphatiques ramifiés. La théorie de la solubilité des polymères et ce modèle n'expliquent donc pas en totalité les différences de composition des hydrocarbures entre les extraits de roche mère et les huiles de réservoir, observées dans les systèmes pétroliers. De même ce modèle n'explique pas la forte accumulation d'hydrocarbures aliphatiques dans les charbons. Ce modèle présente deux limitations : le premier est que les valeurs du coefficient d'avalement volumique sont réparties par classes chimiques mais ne sont pas normalisées. Ainsi le modèle ne respecte pas le principe de conservation de la masse. Ceci engendre des seuils de rétention trop élevés et la somme des compositions est supérieure à 100%. Le deuxième défaut vient du fait que le phénomène d'avalement volumique sous entend un gonflement de la matrice organique. Or ce gonflement à peu de chance de se produire dans des roches soumises à d'importantes surpressions.

Pour conclure, les modèles d'expulsion et de rétention proposés à ce jour mettent en jeu des mécanismes possibles, et sont développés avec plus ou moins de suppositions ce qui conduit à des approches plus ou moins réalistes. En effet la structure du kérogène et la nature des effluents varient avec la maturité de la roche mère, de ce fait craquage thermique et expulsion sont des processus indissociables.

La méthode selon l'invention permet de quantifier la formation et la rétention d'hydrocarbure dans une roche mère à partir d'un nouveau type de simulation. Ce type de simulation est basé sur une technique de modélisation moléculaire dynamique couplée à un champ de forces réactif. Comme pour les modèles mécanistiques, cette approche nécessite comme point de départ une représentation "moléculaire" de la structure du kérogène. La méthode selon l'invention ne nécessite pas l'écriture de centaines de réactions a priori : le schéma réactionnel n'est pas une donnée d'entrée, il devient un résultat de la simulation dynamique. Comme pour les schémas radicalaires, cette nouvelle technique est applicable dans tous les régimes thermiques.

**Description de l'invention**

L'invention concerne une méthode pour quantifier la formation et la rétention d'hydrocarbures au sein d'un système chimique macromoléculaire, comportant la construction d'un modèle moléculaire dudit système au moyen de caractérisations expérimentales d'un échantillon du système, et qui comprend les étapes suivantes :

- on définit un schéma réactionnel de maturation thermique du système chimique macromoléculaire en soumettant ledit modèle moléculaire à une simulation dynamique moléculaire associée à un champ de forces réactif ;
- on quantifie les hydrocarbures formés libres tout au long de la maturation thermique du système macromoléculaire par :

a) détermination de l'évolution physico-chimique du mélange moléculaire défini par ledit schéma réactionnel, par calcul des équilibres de phases, directement à partir de la simulation dynamique moléculaire ;

b) détermination de la cinétique de décomposition thermique du système chimique macromoléculaire par étude cinétique à partir dudit schéma réactionnel.

Dans la méthode selon l'invention, ledit modèle moléculaire et ledit schéma réactionnel sont avantageusement validés par comparaison des résultats issus de ladite simulation dynamique moléculaire réactive avec des mesures expérimentales de maturation thermique dudit système chimique macromoléculaire.

La détermination des équilibres de phases est effectuée de préférence par calcul à chaque instant de l'énergie totale de chaque molécule soumise au champ de forces, puis par bilans successifs pour quantifier la rétention des hydrocarbures dans le système chimique macromoléculaire.

L'étude cinétique est effectuée de préférence par variation de la température du schéma réactionnel.

Dans un mode de réalisation, ledit système chimique macromoléculaire représente la matière organique d'une roche-mère d'un gisement pétrolier.

L'invention concerne également une méthode pour simuler la genèse d'un bassin sédimentaire dans laquelle on modélise la formation et la rétention des hydrocarbures au sein d'une roche mère contenant du kérogène, et dans laquelle l'on réalise les étapes suivantes :

- on définit au moyen de caractérisations expérimentales un modèle moléculaire du kérogène de la roche-mère ;

- on définit un schéma réactionnel en soumettant ledit modèle moléculaire à une simulation dynamique moléculaire associée à un champ de forces réactif ;

- on quantifie l'évolution du kérogène en produits lourds et hydrocarbures, en effectuant une étude cinétique de la décomposition thermique du kérogène à partir dudit schéma réactionnel ;

- on quantifie la rétention d'hydrocarbures au sein de la roche-mère en réalisant des bilans sur les fractions liées à la matrice organique de la roche-mère et sur les fractions libres à partir des équilibres de phase déterminés par la simulation dynamique moléculaire ; et

- on utilise comme données d'entrée d'un simulateur de bassin les quantités d'hydrocarbures formés et les quantités d'hydrocarbures non retenus au sein de la roche-mère, pouvant être expulsés de la roche mère et migrés jusqu'à un réservoir pétrolier.

**Présentation sommaire des figures**

Les caractéristiques et avantages de la méthode selon l'invention, apparaîtront plus clairement à la lecture de la description ci-après d'un exemple non limitatif de réalisation, en se référant aux dessins annexés où :

- La figure 1 montre le principe de la méthode en fonction des différentes échelles d'espace et de temps des phénomènes modélisés.

- La figure 2 illustre l'enchaînement des étapes de la méthode pour parvenir à l'évaluation des hydrocarbures formés et retenus dans la roche-mère.

- La figure 3 illustre le protocole de caractérisation moléculaire du lignite.

- La figure 4 illustre le résultat des simulations dynamiques réactives selon l'invention sur trois modèles moléculaires de fragments de lignite.

- La figure 5 illustre les mécanismes de thermolyse du lignite.

- La figure 6 représente une vue en coupe d'un champ d'exploitation pétrolière.

**Description détaillée**

L'objectif de la méthode selon l'invention est de modéliser numériquement la maturation thermique de la matière organique de la roche -mère ou de tout autre système macromoléculaire, et la rétention des produits de cette réaction dans la matrice organique résiduelle et d'extrapoler éventuellement les résultats à l'échelle du bassin.

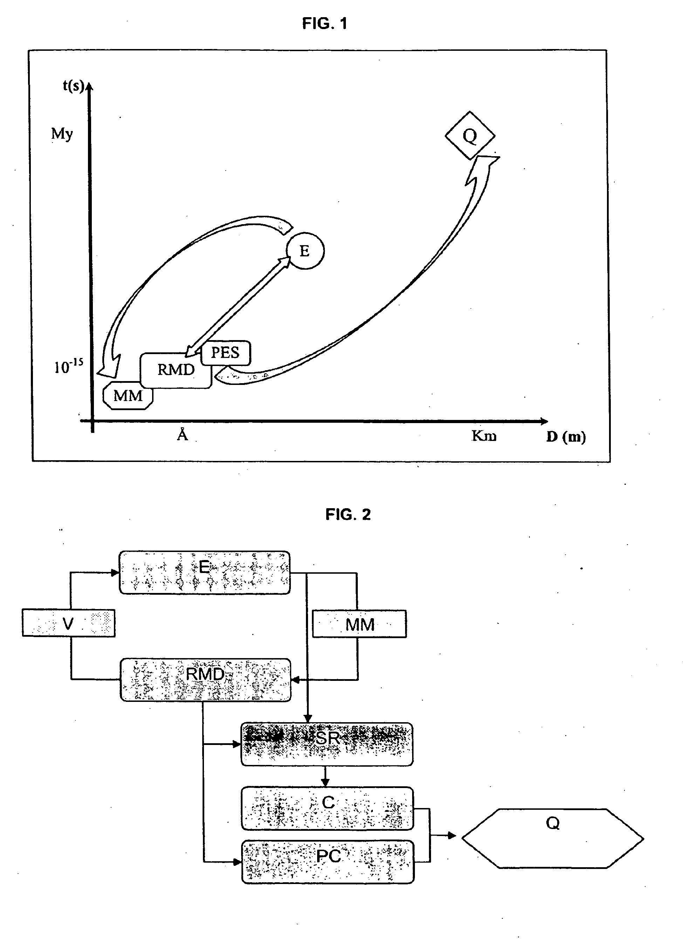

La figure 1 montre le principe de la méthode en fonction des différentes échelles d'espace (D (m): Distance en mètre) et de temps (t(s): temps en seconde). L'échantillon de kérogène initial (ou de toute autre structure macromoléculaire) (E) est caractérisé expérimentalement permettant la détermination d'un modèle moléculaire de la structure (MM (Å, $10^{-15}$s)). Ce modèle moléculaire est la donnée d'entrée des simulations dynamiques couplées à un champ de forces réactif (RMD (Å, $10^{-15}$s)). Ces simulations modélisent la réaction de craquage thermique du modèle moléculaire dans les conditions données, et sont validées par comparaison avec les données expérimentales de maturation thermique. A plusieurs stades de la réaction simulée, des calculs d'équilibres de phases (PES ($\mu$m, $10^{-15}$s)) sont effectués en vue d'établir un bilan physico-chimique du milieu réactionnel. A partir de l'ensemble de ces calculs les hydrocarbures libres et retenus dans le résidu solide sont estimés à l'échelle moléculaire puis extrapolés dans les conditions géologiques (Q (Km, Ma)).

La méthode peut être décomposée en trois étapes majeures :

1- Détermination de la nature et de la structure moléculaire du système chimique macromoléculaire (par exemple kérogène)

2- Simulation moléculaire dynamique réactive

3- Étude thermodynamique et étude cinétique

Ces étapes sont schématisées sur la figure 2 : à partir de données de laboratoires de l'échantillon (E) est établi un modèle moléculaire de l'échantillon (MM) qui est introduit dans une simulation dynamique réactive (RMD). Les résultats de la simulation dynamique réactive sont validés (V) par comparaison avec les données expérimentales. La simulation dynamique réactive (RMD) permet de construire un schéma réactionnel (SR) et de décrire l'évolution physico-chimique de la rétention (PC) à partir de la détermination des équilibres de phases. A partir du schéma réactionnel est déterminée la cinétique de la réaction de décomposition thermique, notamment par craquage thermique (C). Le couplage de l'étude cinétique et de l'étude physico-chimique permet de quantifier les hydrocarbures produits et les hydrocarbures "libres" (Q).

**Première étape : Détermination de la structure moléculaire et de la nature de la matière organique - Etablissement d'un modèle moléculaire**

La première étape de cette méthode est de déterminer la structure et la nature du système macromoléculaire (notamment matière organique de la roche-mère) qui serviront de données de départ à la modélisation de la réaction de maturation thermique. Par exemple, de nombreuses techniques de caractérisation de la matière organique telles que l'analyse élémentaire (AE), la spectroscopie infra rouge à transformée de Fourrier (FT-IR), la spectroscopie à résonance magnétique nucléaire (NMR) et la diffraction des rayons X (DRX) peuvent être utilisées afin de quantifier les différentes fonctions et de connaître l'organisation spatiale de la structure moléculaire. De même, les techniques de caractérisation indirecte telles que l'étude des produits de pyrolyses sont aussi un moyen efficace de détermination de structures macromoléculaires. L'utilisation de modèle moléculaire tel que Signature (générateur aléatoire de macromoléculaire, Faulon 1991 et 2001) associée aux données expérimentales de caractérisation peut permettre d'obtenir un système macromoléculaire plus réaliste. La première étape de la méthode selon l'invention permet l'établissement d'un modèle moléculaire du système macromoléculaire.

**Deuxième étape : Simulation moléculaire dynamique réactive**

La réaction de craquage du modèle moléculaire du système macromoléculaire est reproduite par des calculs de simulation moléculaire dynamique couplés à un champ de forces réactif (RMD). Ces simulations sont comparées à des données expérimentales de maturation thermique (données quantitatives (conversion, bilan de masse, analyse élémentaire...) et qualitatives (caractérisations des produits (par chromatographie gazeuse couplée à un spectromètre de masse GC-MS, résonance magnétique NMR, spectroscopie infra rouge à transformée de Fourrier FT-IR, diffraction des rayons X DRX...) de pyrolyses en milieu ouvert ou fermé ou d'autres expériences de maturation thermique suivant les conditions des simulations). La concordance des résultats expérimentaux et numériques valide à la fois le modèle moléculaire de l'échantillon initial et le mécanisme réactionnel proposé par la simulation.

Les simulations dynamiques sont des modélisations moléculaires en trois dimensions qui permettent de représenter un ensemble de molécules dans un champ de forces décrivant l'état énergétique du milieu. Dans un volume donné un système de molécules possédant n atomes et m liaisons se transformera en un système conservant un nombre n d'atomes mais évoluant vers un nombre m' (différent ou égal à m) de liaisons.

Van Duin et al. (2001) proposent un champ de forces réactif appelé « Reaction Force Field » (*ReaxFF*). Ce champ de forces constitue une approche intermédiaire entre les modèles quantiques et les champs de forces non réactifs.

Les deux particularités de ce champ sont que sa fonction énergétique est basée sur l'ordre des liaisons entre atomes, et qu'il y a continuité entre les énergies liantes et les énergies non liantes. Il en résulte une fonction énergétique assez complexe qui incorpore les contributions partielles d'énergies permettant de décrire les différents types de liaisons ainsi que les ruptures et les formations de liaisons:

$$E_{total} = E_{bond} + E_{over} + E_{under} + E_{val} + E_{pen} + E_{tors} + E_{conj} + E_{vdw} + E_{coulomb}$$

Avec :

$E_{bond}$ = énergie de liaison

$E_{over}$ = énergies de sur-coordination

$E_{under}$ = énergies de sous-coordination

$E_{val}$ = énergie de valence

$E_{pen}$ = terme de pénalité (associé à $E_{over}$, $E_{under}$ dans le cas des allènes)

$E_{tor}$ = énergie de torsion

$E_{conj}$ = énergie de conjugaison

$E_{vdw}$ = énergie de van der Waals

$E_{coulomb}$ = énergie de Coulomb

A chaque terme énergétique correspond une fonction analytique paramétrée reliant l'énergie à l'ordre des liaisons impliquées. Par exemple, l'énergie de liaison entre deux atomes *i* et *j* s'écrit:

$$E_{bond} = -D_e . BO_{ij} . \exp\left(p_{be,1}\left(1 - BO_{ij}^{p_{be,1}}\right)\right)$$

où $BO_{ij}$ est l'ordre de la liaison entre *i* et *j*, $D_e$ et $p_{be,1}$ sont des paramètres calibrés pour différents couples d'atomes. Suivant le principe de Pauling, l'ordre de liaison est lui-même une fonction de la distance relative des atomes. Dans le cas de systèmes pluri atomiques le calcul de $BO_{ij}$ entre deux atomes tient compte de l'environnement atomique. Van Duin et al. (2001) ont développé un mécanisme de calcul liant les ordres de liaison aux positions relatives des atomes dans l'espace en tenant compte de l'ensemble de ces interactions.

A ce jour *ReaxFF* permet de réaliser des simulations dynamiques sur des systèmes constitués d'au moins un des atomes suivants : C, H, O, N, S, Si, Pt, Zr, Ni, Au, V, Bi, Ti, Mo. Les paramètres associés aux différents termes énergétiques sont calibrés par inversion numérique à la fois de données expérimentales (enthalpie de réactions généralement), et de données théoriques calculées par des méthodes quantiques.

Concernant la simulation dynamique en tant que telle, *ReaxFF* se présente sous la forme d'un logiciel incluant à la fois le champ de forces (sous la forme d'un fichier auxiliaire) et le moteur de la dynamique moléculaire. Ce moteur reprend les principes de base de la simulation dynamique, dans lesquelles le mouvement des atomes est décrit par la mécanique newtonienne dans l'espace à trois dimensions :

$$\begin{cases} \vec{F}_i = m_i \dfrac{d^2\vec{r}_i(t)}{dt^2} = -\dfrac{d\vec{E}}{dr_i(t)} \\ \vec{E} = f(BO_{ij}) = f'(r_1,...,r_n) \end{cases}$$

Avec :

$F_i$ = force s'exerçant sur l'atome $i$

$r_i$ = trajectoire de l'atome $i$

Ainsi les simulations dynamiques avec le champ de force *ReaxFF* permettent de reproduire les mécanismes intra et intermoléculaires de réactions chimiques.

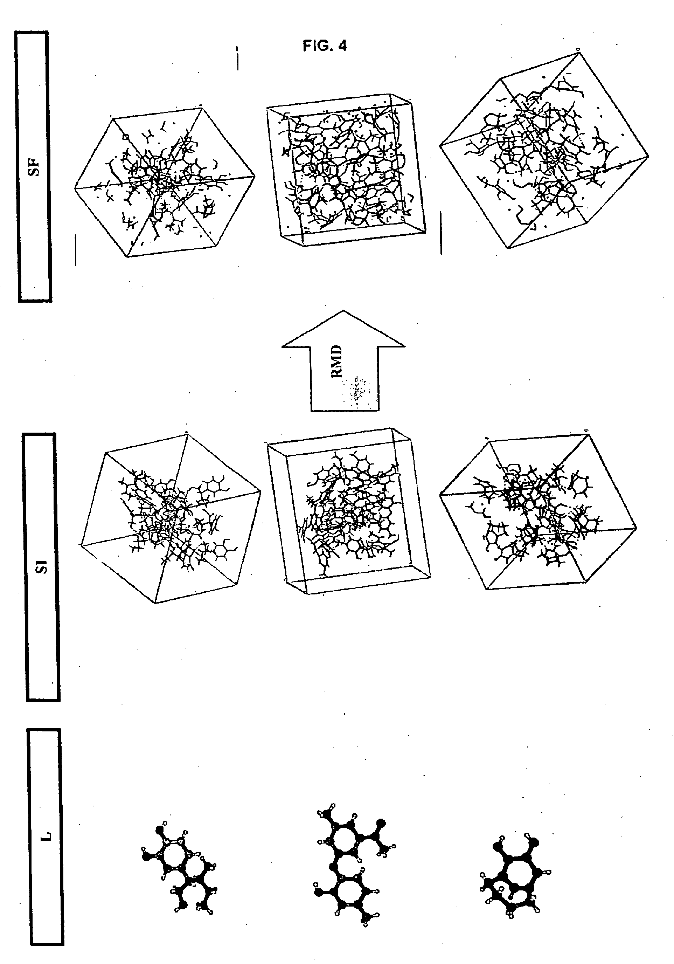

La figure 4 illustre les paramètres principaux des simulations dynamiques réactives sur trois fragments de lignite. Dans un premier temps (SI) une ou plusieurs molécules ($N_0$ atomes et $M_0$ liaisons atomiques) sont construites en trois dimensions dans une boîte de dimensions prédéfinies. Il est possible de faire varier les paramètres de volume de la boîte, pression et/ou température pendant l'intégration du modèle en fonction du temps. On obtient une succession d'états du système (SF) évoluant en fonction du temps dans le champ de forces ($N_0$ atomes mais $M_t$ ($\neq M_0$) liaisons).

**Troisième et quatrième étapes : étude thermodynamique et étude cinétique**

Les résultats des simulations dynamiques permettent d'établir un schéma réactionnel et peuvent être interprétés ou développés suivant deux approches:

- approche physico-chimique de la réaction par l'étude des équilibres des phases : étude thermodynamique,
- approche cinétique de la réaction de décomposition pour quantifier la formation des produits majoritairement formés ou la dissociation du kérogène modèle de départ : étude cinétique.

*Étude physicochimique* (PC) : à chaque pas de temps, la simulation dynamique calcule l'énergie totale de chaque molécule du champ de forces. A partir de ces énergies, il est possible de trier les molécules suivant leur état physique (liquide, solide ou gazeux). On peut alors suivre l'évolution physique de chaque molécule au cours de la simulation de la réaction. Les bilans successifs d'équilibres de phases (PES) permettent de suivre l'évolution physico-chimique de la maturation thermique de l'échantillon étudié. Cette analyse thermodynamique permet d'étudier et de quantifier les produits de thermolyse qui sont indépendants du « résidu » (une ou plusieurs molécules en phase solide et de masse moléculaire élevée) des produits dépendants.

*Étude cinétique* (C) : l'étude cinétique de la réaction nécessite des calculs dynamiques à différentes températures. A chaque température, des bilans quantitatifs d'apparition ou de disparition de molécules majoritaires sont établis pour calculer la vitesse de la réaction (k:vitesse d'apparition ou de disparition d'un espèce chimique). A partir des valeurs de différentes vitesses de réactions de chaque espèce chimique aux différentes températures, on peut alors calculer les paramètres cinétiques (énergie d'activation, $E_a$ et facteur pré exponentiel, A) et en déduire la cinétique de décomposition thermique de chaque espèce moléculaire sur la gamme de température étudiée.

Finalement à partir d'une double étude (physicochimique et cinétique) des mécanismes proposés par les simulations dynamiques, les hydrocarbures libres (liquides et gazeux), non retenus dans le résidu solide, sont quantifiables tout au long de la maturation numérique de l'échantillon. Ils représentent, dans les bassins sédimentaires, les hydrocarbures susceptibles d'être expulsés de la roche mère (Q) et peuvent ainsi constituer un paramètre d'entrée pour les simulateurs de bassins.

**Exemple : étude du charbon lignitique:**

Le charbon Brun d'Australie de cet exemple est constitué pour l'essentiel de molécules de lignite (L). Certaines caractéristiques de ce charbon, ainsi que des structures chimiques proches ont été proposées dans les travaux de Hatcher 1988 .

*Définition du système chimique de départ : modèle moléculaire*

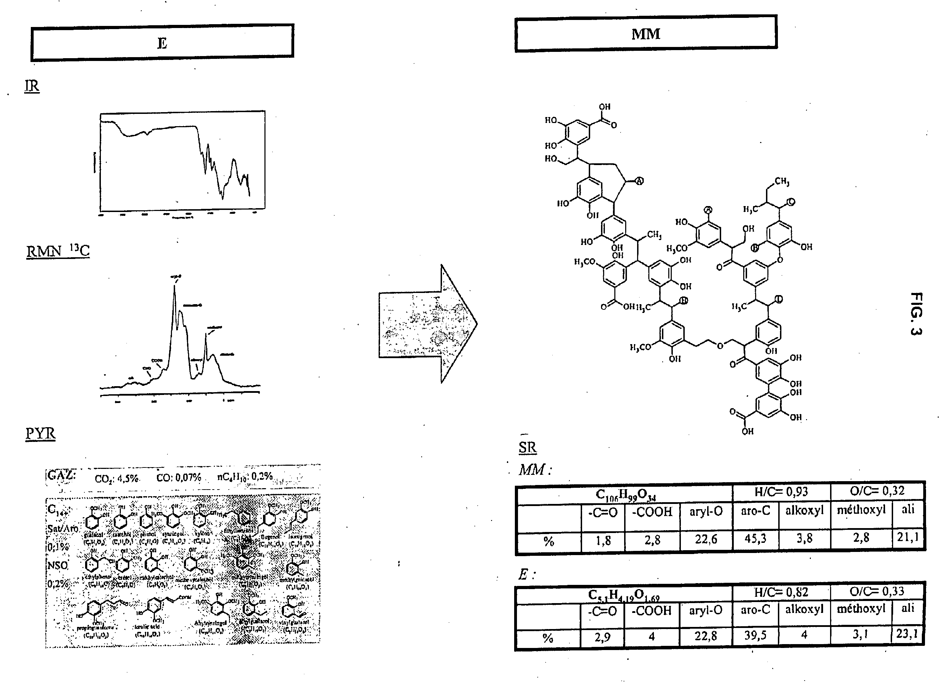

Dans un premier temps, à partir de mesures expérimentales de caractérisation de la matière organique réalisées sur un échantillon de ce charbon (analyse RMN, spectroscopie infrarouge IR, composition atomique, pyrolyses en système fermé PYR-Figure 3), une identification et un comptage des fonctions chimiques ont été effectués puis, par comparaison avec les structures de lignites proposées par Hatcher (Hatcher, 1988) , il en est déduit que le charbon peut être correctement représenté comme un agglomérat d'une forme de lignite qu'on modélise par une macromolécule d'environ 250 atomes. Dans cet exemple le modèle de kérogène est construit manuellement. Pour des kérogènes plus complexes, des simulateurs tels que Signature (Faulon 1991, 2001) peuvent être utilisés pour organiser de façon aléatoire des motifs connus de la structure.

*Modélisation de la réactivité thermique : simulation dynamique réactive (RMD)*

Une fois le système chimique de départ défini, on modélise dans un deuxième temps la réactivité thermique de tout ou partie de la structure chimique déduite dans l'étape précédente. On utilise pour cela un algorithme de simulation dynamique moléculaire associé à un champ de forces particulier, appelé *ReaxFF* (van Duin et al., 2001) permettant de prédire, entre autres le craquage thermique de matière organique dans des conditions données. Des exemples de telles simulations sont montrés dans la Figure 4. Dans ces exemples, nous avons modélisé le craquage thermique de trois fragments typiques de la structure de lignite proposée dans la Figure 3. Pour chacun des trois fragments de lignite (L) sélectionnés :

Nombre d'unités: 15
Volume: 3723.2 $Å^3$
Densité: 1.4 kg.$L^{-1}$

Nombre d' unités: 17
Volume: 5661.2$Å^3$
Densité < 1.4 kg.$L^{-1}$

Nombre d'unités: 21
Volume: 4870.9 $Å^3$
Densité < 1.4 kg.$L^{-1}$

Pour chaque fragment ces simulations sont réalisées de la manière suivante :

- on construit avec l'algorithme un volume contenant n motifs du même fragment

- après une étape d'initialisation (équilibrage des vélocités des atomes et calcul de la densité du système à P et T expérimentales), les simulations dynamiques réactives sont effectuées sous différentes conditions thermiques et à volume constant. Dans la Figure 4 , la température T est supérieure à 2000 K. Bien que la durée physique modélisée soit au final de l'ordre de quelques picosecondes, les temps de calcul peuvent être très longs (quelques heures à quelques jours CPU).

A la fin d'un calcul, dans les systèmes finaux (SF), le volume contient de nouvelles molécules issues des différents mécanismes de décomposition thermique ayant affecté les fragments de lignite initiaux (systèmes initiaux SI). Le logiciel permet l'identification et le comptage de l'ensemble des molécules présentent dans le volume à tout instant. L'analyse fine de ces données permet donc à la fois de prédire la composition des produits de craquage des fragments de départ, et de déterminer les mécanismes qui en sont à l'origine.

*Bilan des résultats des simulations dynamiques réactives : obtention d'un schéma réactionnel*

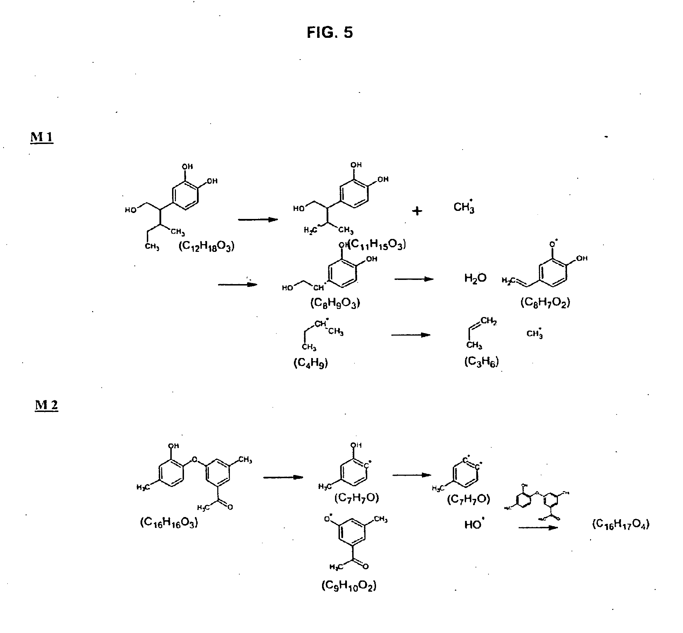

Dans un troisième temps, on fait donc un bilan des résultats des simulations dynamiques réactives, afin d'en extraire un schéma général de décomposition thermique du charbon. Dans l'exemple donné, ce travail montre (Figure 5) qu'une majeure partie des produits de pyrolyse observés expérimentalement est obtenue au moyen de deux mécanismes principaux de craquage affectant la lignite. Ces deux réactions expliquent à la fois la formation d'eau et de méthane, par un processus de défonctionalisation (mécanisme 1 - M1), et la dépolymérisation (mécanisme 2 - M2) par étapes de la lignite, à l'origine des constituants de la fraction $C_{14+}$ illustrée dans la figure 3.
On en conclut qu'un schéma général de pyrolyse du charbon étudié devrait avoir la forme suivante :

- la lignite subit une dépolymérisation en fragments;

- lesdits fragments subissent des défonctionnalisations pour donner des molécules telles que des molécule d'eau (H2O), de méthane (CH4) et d'autres hydrocarbures.

*Étude cinétique*

Dans le but de quantifier la thermolyse du lignite, une étude cinétique est effectuée à partir du schéma réactionnel prédit par la modélisation dynamique réactive

En règle générale (Tissot 1969, Braun et Burnham 1989 et 1990, Pepper et Corvi 1995), les modèles de craquage primaire correspondent à une série de réactions simultanées, indépendantes et compétitives, décrivant le transfert de masse entre le kérogène source et les hydrocarbures. Un exemple de schéma réactionnel est représenté ci-dessous. A chaque réaction du schéma correspond une loi de vitesse d'ordre 1 qui dépend à la fois du temps et de la température. Ainsi, la quantité m d'hydrocarbures formée au cours du temps s'exprime par :

$$\begin{cases} m = X_{inf} \sum_{i=1}^{N} X_i q_i \\ \dfrac{d q_i}{dt} = k_i (1 - q_i)^n \end{cases} \qquad (1)$$

avec : $q_i$ = taux de conversion de la réaction i
$X_i$ = contribution relative de la réaction i
$X_{inf}$ = potentiel pétrolier
$k_i$ = constante de vitesse de la réaction i
n = ordre de la réaction

$X_i$ et $k_i$ obéissent aux lois suivantes :

$$\sum_{i=1}^{N} X_i = 1 \qquad (2)$$

$$k_i = A_i \exp\left(-\frac{E_i}{RT}\right) \qquad (3)$$

avec $A_i$ = facteur de fréquence et $E_i$ = énergie d'activation pour la réaction i.

*Étude thermodynamique des molécules*

A partir du schéma réactionnel des simulations dynamiques, il est aussi possible de trier les molécules formées suivant leur poids moléculaire et d'en déterminer leur état thermodynamique. Des simulateurs utilisant la méthode de Monte Carlo permettent l'étude théorique des équilibres de phases dans un mélange moléculaire. L'utilisation de cette méthode ou d'une méthode équivalente sur le mélange moléculaire (produits et réactifs de la thermolyse du lignite) permet de suivre l'évolution thermodynamique de la réaction dans le temps. Cette analyse thermodynamique permet d'étudier et de quantifier les produits de thermolyse qui sont indépendants du « résidu » (une ou plusieurs molécules en phase solide et de masse moléculaire élevée) des produits dépendants.

Les potentialités de la modélisation moléculaire permettent de produire des modèles moléculaires complexes (cas du kérogène), de prédire les propriétés thermodynamiques à l'équilibre de ces structures chimiques, mais aussi de calculer des paramètres impliqués dans la rétention, tels que la viscosité, la diffusion, la densité, l'état des phases, la répartition des charges électrostatiques, les forces liantes et repoussantes et de prédire la réactivité thermique de ces structures en fonction de la pression et de la température, et de déterminer la nature chimique des produits formés.

La méthode selon l'invention utilise une simulation dynamique réactive, ce qui permet non seulement de prédire l'évolution du kérogène en produits lourds et hydrocarbures, mais aussi de réaliser des bilans sur les fractions liées à la matrice organique et sur les fractions libres, donc de quantifier la rétention.

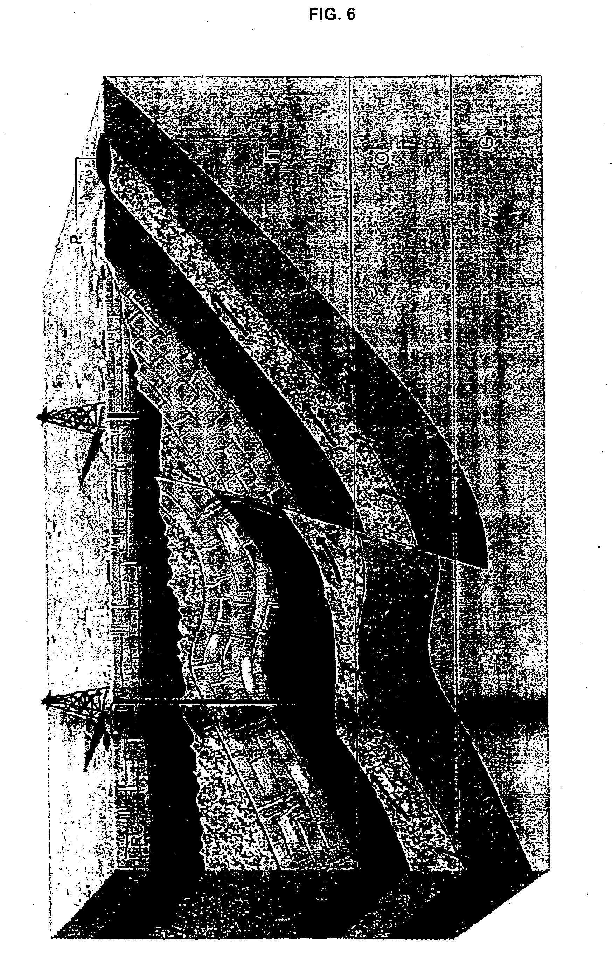

La méthode servant à modéliser la réactivité thermique de systèmes chimiques complexes a été appliquée à la quantification de la formation et la rétention d'hydrocarbures au sein d'un système carboné complexe, tel que la matière organique contenue dans une roche mère. La figure 6 représente un exemple de système d'exploration pétrolière. La zone G représente la fenêtre à gaz (généralement température supérieure à 150°C et pression supérieure à 53 MPa). La zone O représente la fenêtre à huile (température généralement comprise entre 80°C et 150°C, pression comprise entre 24 MPa et 53 MPa). La zone IT est la zone d'immaturité thermique (température inférieure à 80°C et pression inférieure à 24 MPa). Les hydrocarbures "libres" migrent de la roche-mère (RM) vers la zone réservoir (RR). En l'absence de roche couverture (RC) en surface, le pétrole (P) peut suinter en surface. Les forages traversent les couches de roche pour prélever le brut dans la roche réservoir (RR).

On peut également envisager des applications de cette modélisation dans d'autres domaines particuliers afin de comprendre et/ ou de quantifier un processus multi échelle, simulable par l'expérience et s'expliquant par des mécanismes chimiques complexes et/ou inconnus. On peut citer comme exemples non limitatifs le comportement thermique des bruts lourds pendant une injection de vapeur ou d'air chaud dans un réservoir, et l'hydrocracking des asphalthènes en raffinage.

## Revendications

1.  Méthode pour quantifier la formation et la rétention d'hydrocarbures au sein d'un système chimique macromoléculaire, comportant la construction d'un modèle moléculaire dudit système au moyen de caractérisations expérimentales d'un échantillon du système, **caractérisée en ce qu'**elle comprend les étapes suivantes :

    - on définit un schéma réactionnel de maturation thermique du système chimique macromoléculaire en soumettant ledit modèle moléculaire à une simulation dynamique moléculaire associée à un champ de forces réactif ;
    - on quantifie les hydrocarbures formés libres tout au long de la maturation thermique du système macromoléculaire par :

        a) détermination de l'évolution physico-chimique du mélange moléculaire défini par ledit schéma réactionnel, par calcul des équilibres de phases, directement à partir de la simulation dynamique moléculaire ;
        b) détermination de la cinétique de décomposition thermique du système chimique macromoléculaire par étude cinétique à partir dudit schéma réactionnel.

2.  Méthode selon la revendication 1, dans laquelle ledit modèle moléculaire et ledit schéma réactionnel sont validés par comparaison des résultats issus de ladite simulation dynamique moléculaire réactive avec des mesures expérimentales de maturation thermique dudit système chimique macromoléculaire.

3.  Méthode selon l'une des revendications 1 ou 2 dans laquelle la détermination des équilibres de phases est effectuée par calcul à chaque instant de l'énergie totale de chaque molécule soumise au champ de forces, puis par bilans successifs pour quantifier la rétention des hydrocarbures dans le système chimique macromoléculaire.

4.  Méthode selon l'une des revendications 1 à 3 dans laquelle l'étude cinétique est effectuée par variation de la température du schéma réactionnel.

5.  Méthode selon l'une des revendications précédentes, dans laquelle ledit système chimique macromoléculaire représente la matière organique d'une roche-mère d'un gisement pétrolier.

6.  Méthode pour simuler la genèse d'un bassin sédimentaire dans laquelle on modélise la formation et la rétention des hydrocarbures au sein d'une roche mère au moyen de la méthode selon la revendication 5, dans laquelle :

    - le système chimique macromoléculaire est du kérogène contenu dans la roche-mère ;
    - on quantifie l'évolution du kérogène en produits lourds et hydrocarbures,
    - on quantifie la rétention d'hydrocarbures au sein de la roche-mère en réalisant des bilans sur les fractions liées à la matrice organique de la roche-mère et sur les fractions libres à partir des équilibres de phase; et
    - on utilise comme données d'entrée d'un simulateur de bassin les quantités d'hydrocarbures formés et les quantités d'hydrocarbures non retenus au sein de la roche-mère, pouvant être expulsés de la roche mère et migrés jusqu'à un réservoir pétrolier.

**Claims**

1. Method for quantifying the formation and retention of hydrocarbons within a macromolecular chemical system, comprising the construction of a molecular model of said system using experimental characterisations of a sample of the system, **characterised** that it includes the following steps:

   - a reaction scheme for the thermal maturation of the macromolecular chemical system is defined by subjecting the said molecular model to a molecular dynamic simulation associated with a field of reactive forces;
   - the hydrocarbons formed in the free state during thermal maturation of the macromolecular system are quantified by:

     a) determining the physical and chemical changes in the molecular mixture defined by the said reaction scheme, through calculating phase equilibria, directly from the molecular dynamic simulation,
     b) determining the thermal decomposition kinetics of the macromolecular chemical system by kinetic investigation based on the said reaction scheme.

2. Method according to Claim 1, in which the said molecular model and the said reaction scheme are validated by comparing the results obtained from the said reactive molecular dynamic simulation with experimental measurements of thermal maturation of the said macromolecular chemical system.

3. Method according to one of Claims 1 or 2, in which phase equilibria are determined by calculating at each instant the total energy of each molecule subjected to the field of forces, then by successive balances to quantify hydrocarbon retention in the macromolecular chemical system.

4. Method according to one of Claims 1 to 3, in which the kinetic investigation is performed by varying the temperature of the reaction scheme.

5. Method according to one of the preceding Claims, in which the said macromolecular chemical system represents the organic matter in a source rock for an oil field.

6. Method for simulating the genesis of a sedimentary basin in which the formation and retention of hydrocarbons within a source rock is modelled using the method according to Claim 5, in which:

   - the macromolecular chemical system is kerogen present in the source rock,
   - change of the kerogen into heavy products and hydrocarbons is quantified,
   - hydrocarbon retention within the source rock is quantified by preparing balances for the fractions bound to the organic matrix of the source rock and free fractions based on the phase equilibria, and
   - the quantities of hydrocarbons formed and the quantities of hydrocarbons not retained within the source rock, which may be expelled from the source rock and which might migrate to an oil reservoir, are used as input data to a basin simulator.

**Patentansprüche**

1. Verfahren zum Quantifizieren der Bildung und der Retention von Kohlenwasserstoffen innerhalb eines makromolekularen chemischen Systems, das die Konstruktion eines molekularen Modells des Systems mittels experimenteller Charakterisierungen einer Probe des Systems umfasst, **dadurch gekennzeichnet, dass** es die folgenden Schritte umfasst:

   - Definieren eines Reaktionsschemas der thermischen Reifung des makromolekularen chemischen Systems, indem das molekulare Modell einer molekularen dynamischen Simulation unterworfen wird, die mit einem Reaktionskraftfeld assoziiert ist;
   - Quantifizieren der Kohlenwasserstoffe, die während der thermischen Reifung des makromolekularen Systems frei gebildet wurden, durch:

     a) Bestimmen der physikalisch-chemischen Entwicklung des molekularen Gemischs, das durch das Reaktionsschema definiert ist, durch Berechnen der Phasengleichgewichte direkt ausgehend von der molekularen dynamischen Simulation;

b) Bestimmen der Kinetik des thermischen Abbaus des makromolekularen chemischen Systems durch kinetische Untersuchung ausgehend von dem Reaktionsschema.

2. Verfahren nach Anspruch 1, wobei das molekulare Modell und das Reaktionsschema durch den Vergleich der Ergebnisse, die aus der molekularen dynamischen Reaktionssimulation stammen, mit experimentellen Messungen der thermischen Reifung des makromolekularen chemischen Systems validiert werden.

3. Verfahren nach einem der Ansprüche 1 oder 2, wobei die Bestimmung der Phasengleichgewichte durch das Berechnen zu jedem Zeitpunkt der Gesamtenergie jedes Moleküls, das dem Kraftfeld unterworfen wird, dann durch sukzessives Bilanzieren, um die Retention der Kohlenwasserstoffe in dem makromolekularen chemischen System zu quantifizieren, durchgeführt wird.

4. Verfahren nach einem der Ansprüche 1 bis 3, wobei die kinetische Untersuchung durch Variation der Temperatur des Reaktionsschemas durchgeführt wird.

5. Verfahren nach einem der vorhergehenden Ansprüche, wobei das makromolekulare chemische System das organische Material eines Muttergesteins eines Erdölvorkommens darstellt.

6. Verfahren zum Simulieren der Genese eines Sedimentationsbeckens, wobei die Bildung und die Retention von Kohlenwasserstoffen innerhalb eines Muttergesteins mittels des Verfahrens nach Anspruch 5 modelliert werden, wobei:

- das makromolekulare chemische System Kerogen ist, das in dem Muttergestein enthalten ist;
- die Entwicklung der Kerogene zu Schwerprodukten und Kohlenwasserstoffen quantifiziert wird,
- die Retention von Kohlenwasserstoffen innerhalb des Muttergesteins quantifiziert wird, indem Bilanzen an den Fraktionen, die mit der organischen Matrix des Muttergesteins verbunden sind, und an den freien Fraktionen, ausgehend von den Phasengleichgewichten, erstellt werden; und
- als Eingangsdaten eines Beckensimulators die gebildeten Kohlenwasserstoffmengen und die Mengen an Kohlenwasserstoffen verwendet werden, die nicht innerhalb des Muttergesteins zurückgehalten wurden, die aus dem Muttergestein ausgetrieben und bis zu einer Erdöllagerstätte migriert werden können.

## FIG. 1

## FIG. 2

## FIG. 3

# FIG. 4

FIG. 5

## FIG. 6

**EP 1 884 620 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **BEHAR F ; VANDENBROUCKE M ; TANG Y ; MARQUIS F ; ESPITALIÉ J.** Thermal cracking of kerogen in open and closed systems: determination of kinetic parameters and stoichiometric coefficients for oil and gas generation. *Org. Geochem,* 1997, vol. 26 (5-6), 321-339
- **BURNHAM, A. K ; BRAUN, R. L.** Development of detailed model of petroleum formation, destruction, and expulsion from lacustrine and marine source rocks. *Advances in Organic Geochemisrty,* 1989, vol. 16 (1-3), 27-39
- **BURNHAM, A. K ; BRAUN, R. L.** Mathematical model of oil generation, degradation, and expulsion. *Energy and Fuel,* 1990, vol. 4, 132-146
- Prediction Elucidation and Molecular Modeling. Algorithms and Applications in Geochemistry. **FAULON, J. L.** Ph. D.Thesis. 1991
- **FAULON, J. L.** Stochastic Generator of Chemical Structure. *Building Polymeric Systems with Specified Properties, J. Comput. Chem,* 2001, vol. 22 (4), 580-590
- Predicting oil and gas compositional yields via chemical structure- chemical yield modeling (CS-CYM. **FREUND, H ; WALTERS, C. C ; KELEMEN, S. R ; SISKIN, M ; CURRY, D. J ; XIAO, Y ; OLMSTEAD, W. N ; GORBATY, M. L ; BENCE, A. E.** Organic Geochemistry: Challenges for the 21st Century. 2005, vol. 1, 66-67
- **HATCHER, P. G.** Dipolar-Dephasing 13C studies of decomposed wood and coalified xylem tissue: Evidence for chemical structural changes associated with defunctionalization of lignin structural units during coalification. *Energy & Fuels,* 1988, vol. 2, 48-58
- Estimating the petroleum expulsion behaviour of source rocks: a novel quantitative approach. **PEPPER, A. S.** In Petroleum Migration. Geological Society, 1991, vol. 59, 9-31
- **PEPPER, A. S ; CORVI, P. J.** Simple kinetic models of petroleum formation. PartIII: Modelling and open system. *Marin and Petroleum Geology,* 1995, vol. 12 (4), 417-452
- **PEPPER, A. S ; DODD, T. A.** Simple models of petroleum formation. Part II: oil to ges cracking. *Mar. Petrol. Geol,* 1995, vol. 12, 321-340
- **RITTER, U.** Fractionation of petroleum during expulsion from kerogen. *Journal of Geochemical Exploration,* 2003, vol. 78-79, 417-420
- **RITTER, U.** Solubility of petroleum compounds in kerogen: implications for petroleum expulsion. *Organic Geochemistry,* 2003, vol. 34, 319-326
- **Ritter, U. ; Groever, A.** Adsorption of petroleum compounds in vitrinite: implications for petroleum expulsion from coal. *International Journal of Coal Geology.,* 2005, vol. 62 (3), 183-191
- **TISSOT, B.** *Revue Inst. Fr. Pétrole,* 1969, vol. 24 (4), 470-501
- **UNGERER, P.** State of the art of research in kinetic modelling of oil formation and expulsion. *Advances in organic goechemistry, Organic Geochemistry,* 1989, vol. 16 (1-3), 1-25
- **VAN DUIN A.C.T ; SIDDHARTH D ; LORANT F ; GODDARD III W.A.** ReaxFF: a reactive force field for hydrocarbons. *J. Phys. Chem. A,* 2001, vol. 105, 9396-9409